# EUROPEAN PATENT SPECIFICATION

(11) **EP 0 561 779 B1**
(45) Date of publication and mention of the grant of the patent: **28.06.1995**
(21) Application number: 91900226.1
(22) Date of filing: 13.12.1990
(51) Int. Cl.: C07C 11/02

(54) **PROCESS FOR PRODUCING 1-OCTENE**
VERFAHREN ZUR HERSTELLUNG VON 1-OKTEN
PROCEDE DE PRODUCTION DE 1-OCTENE

(43) Date of publication of application: 29.09.1993
(73) Proprietor: THE DOW CHEMICAL COMPANY (a Delaware corporation), Midland Michigan 48640 (US)
(72) Inventor: BOHLEY, Robert, Christian, NL-4537 CJ Terneuzen (NL); JACOBSEN, Grant, Berent, NL-4542 BM Hoek (NL); PELT, Hendrik, Leendert, NL-4535 HC Terneuzen (NL); SCHAART, Barend, Johannes, NL-4535 CL Terneuzen (NL); SCHENK, Michiel, NL-4576 BB Koewacht (NL); VAN OEFFELEN, Dominicus, Anthonius, Gerardus, NL-4532 JR Terneuzen (NL)
(74) Representative: Smulders, Theodorus A.H.J., Ir.
(86) International application number: EP9002194
(87) International publication number: WO9210450

(56) References cited:
- Chemical Abstracts, volume 114, 7 January 1991, page 574, abstract no. 5796w, Columbus, Ohio, US; & JP-A-02 172 924

## Description

The present invention relates to a process for producing 1-octene.

1-Octene is used in large amounts as starting material for the production of various industrial products such as, for example, surfactant intermediates, plasticizer alcohols, lubricants and in the production of polymeric materials. For many of the industrial applications it is desirable to have 1-octene in a form as pure as possible, especially in the substantial absence of its branched olefinic C₈-isomers, such as 2-methyl-1-heptene, 2-methyl-2-heptene, 3-methyl heptenes, 2-ethyl-1-hexene, dimethyl hexenes and similar compounds.

The most widely used commercial processes to produce 1-octene comprise oligomerization of ethylene to produce a range of linear α-olefins as major products. By suitable selection of catalyst materials and process conditions the selectivity towards 1-octene can be optimized. However, as illustrated in ALPHA OLEFINS APPLICATIONS HANDBOOK, G.R. Lappin, J.D. Sauer, MARCEL DEKKER, INC. New York, pages 44-49, such oligomerization processes also produce some branched olefinic C₈-isomers, such as 2-ethyl-1-hexene. As such by-product isomers frequently have a boiling point (2-ethyl-1-hexene having a boiling point of 120.0 °C) which is very close to the boiling point of 1-octene (121.3°C), these by-products will be recovered in the 1-octene fraction isolated from the oligomerization reaction product. In view of this small difference in boiling point, it is very difficult to remove such isomers from the main product 1-octene by means of simple distillation techniques. The said isomers could be removed from the 1-octene product by means of adsorption-desorption separation techniques, but these techniques would increase the costs of the 1-octene product substantially and, accordingly, are not used in commercial practice to produce 1-octene. Therefore, the 1-octene processes commercially used generally yield 1-octene having a purity of at the most about 97.5%, whereas representative amounts of branched olefinic C₈-isomers are between 0.5 and 1.5 wt%.

The abstract of JP 02,172,924 in Chemical Abstracts 114 (1991) 5796w describes a process for producing 1-octene comprising the reaction of 1,3-butadiene and water in the presence of a telomerization catalyst with a recommended palladium concentration of 0.0005-0.005 gramatoms palladium per liter reaction mixture. The reaction mixture is required to comprise a solvent which dissolves both water, butadiene and the catalyst components. This solvent needs to be removed from the process at a later stage. The reaction product of water and butadiene is subsequently subjected to hydrogenation providing a 1-substituted octane, which is decomposed in the presence of a suitable catalyst to form 1-octene.

According to the present invention, by a careful selection of starting products and reaction steps, 1-octene containing substantially no branched olefinic C₈-isomers can be obtained.

Accordingly, the present invention provides a process for producing 1-octene which comprises the steps of:
(i) reacting 1,3-butadiene with a primary aliphatic alcohol or aromatic hydroxy compound having formula R-H, in the presence of a telomerization catalyst comprising palladium and a tertiary phosphorous ligand compound, in an amount of less than 0.0001 gramatoms palladium catalyst per mole of 1,3-butadiene, to form a 1-substituted-2,7-octadiene of formula CH₂=CH-CH₂-CH₂-CH₂-CH=CH-CH₂-R, in which R represents the residue of the primary aliphatic alcohol or aromatic hydroxy compound;
(ii) subjecting the 1-substituted-2,7-octadiene formed in step (i) to hydrogenation in the presence of a hydrogenation catalyst to form a 1-substituted octane of formula CH₃-CH₂-CH₂-CH₂-CH₂-CH₂-CH₂-CH₂-R; and
(iii) decomposing the 1-substituted octane formed in step (ii) in the presence of a suitable catalyst to form 1-octene.

The above mentioned reaction steps are illustrated by the following equations:

(i) 2 CH₂=CH-CH=CH₂ + R-H → CH₂=CH-CH₂-CH₂-CH₂-CH=CH-CH₂-R

(ii) CH₂=CH-CH₂-CH₂-CH₂-CH=CH-CH₂-R + 2 H₂ → CH₃-CH₂-CH₂-CH₂-CH₂-CH₂-CH₂-CH₂-R

(iii) CH₃-CH₂-CH₂-CH₂-CH₂-CH₂-CH₂-CH₂-R → CH₃-CH₂-CH₂-CH₂-CH₂-CH₂-CH=CH₂ + R-H

The reaction in step (i) of the process of the present invention between an active hydrogen-containing compound R-H and 1,3-butadiene, also referred to as telomerization reaction, has been known per se for many years.

In step (i) of the present process any suitable telomerization process can be used which yields as primary product the compound of formula CH₂=CH-CH₂-CH₂-CH₂-CH=CH-CH₂-R (1-substituted-2,7-octadiene). Suitable telomerization processes are for example described in US 3499042, US 3518315, US 3530187, GB 1178812, NL-A 6816008, GB 1248593, US 3670029, US 3670032, US 3769352, US 3887627, GB 1354507, DE-A 2040708, US 4142060, US 4219677, US 4146738, US 4196135 and EP-A 218100. The telomerization process may be carried out continuously or batch-wise.
The 1,3-butadiene used in step (i) may be employed as substantially pure butadiene or as a crude C₄-hydrocarbons mixture. Such a crude C₄-mixture contains in general, besides 1,3-butadiene other C₄-hydrocarbons such as butenes and butanes. These other C₄-hydrocarbons do not essentially influence conversion and selectivity of the 1,3-butadiene. Such a crude C₄-hydrocarbon mixture can be produced as a by-product in the pyrolysis of naphtha, gas oil, LPG et cetera for the production of ethylene. As the selectivity and conversion towards the desired telomerization product, based on the 1,3-butadiene present in the crude C₄-hydrocarbons mixture, are about the same as for pure 1,3-butadiene, it is advantageous to use the crude C₄-mixture in step (i) of the present process, since doing so avoids the investments and costs associated with first extracting and purifying the 1,3-butadiene from the crude C₄-mixture. The crude C₄-mixture produced in the cracking of naphtha, gas oil or LPG usually has a 1,3-butadiene content of 20 to 70 % by weight. When the crude C₄-mixture contains acetylenes, it is advantageous to selectively hydrogenate the crude C₄-mixture in order to remove these acetylenes prior to its use in the present process.

The active hydrogen-containing compound R-H employed as co-reactant in step (i) of the process of the invention can be any primary aliphatic alcohol or aromatic hydroxy compound. The functional groups of these types of compounds contain active hydrogen atoms having a high reactivity towards 1,3-butadiene.

Exemplary of such primary aliphatic alcohols are mono- or polyhydric alcohols containing primary OH-groups which alcohols can be linear or branched saturated compounds having up to 20 carbon atoms as well as unsaturated alcohols such as allylalcohol; especially the mono- or polyhydric aliphatic alcohols having up to 8 carbon atoms, such as methanol, ethanol, propanol, butanol, ethylene glycol, propylene glycol, glycerol and the like.

Exemplary of hydroxy-aromatic compounds are aromatic compounds containing one or more rings such as phenol, benzylalcohol, cresols, xylenols, naphtol, as well as polyhydric compounds such as resorcinol, hydroquinone and pyrocatechol. Also alkyl-, alkoxy- and/or halogen-substitued aromatic hydroxy compounds may be used, such as o-methoxyphenol, p-chlorophenol and the like.

In step (i) of the process of the invention, as the active hydrogen-containing compound preferably methanol or ethanol is used.

In general the ratio between the active hydrogen-containing compound R-H and 1,3-butadiene (hereinafter referred to as "reactants ratio" and expressed in moles of R-H per mole of 1,3-butadiene) is higher than 0.5. According to the present invention the active hydrogen-containing compound R-H is used in an amount of preferably 1 to 20 moles, and more preferably 1 to 10 moles per mole of 1,3-butadiene.

The telomerization catalyst to be used in step (i) of the present invention can be any catalyst comprising palladium and a tertiary phosphorous ligand compound which, when contacting the reactants in its presence, promotes the formation of a 1-substituted-2,7-octadiene. The telomerization catalysts include those supported on an inert carrier. Suitable examples of such catalyst compounds for use in step (i) of the process of the present invention are described in an article of A. Behr titled "Homogeneous Transition Metal Catalysts" in Aspects of Homogeneous Catalysis (1984), Vol. 5, pages 3-73, as well as in the references cited hereinbefore. It is preferred that the catalyst is homogeneous under reaction conditions.

Both palladium(II) compounds and palladium(0) complexes may be used in the present process. Examples of suitable palladium(II) compounds include: palladium(II) salts, such as palladium bromide, palladium chloride, palladium formate, palladium acetate, palladium propionate, palladium octanoate, palladium carbonate, palladium sulfate, palladium nitrate, palladium borate, palladium citrate, palladium hydroxide, palladium aryl- or alkylsulfonates, sodium or potassium chloropalladate; compounds of formula PdX₂L₂ in which X is a monovalent acid residue and L is a ligand selected from the group of organic nitriles, such as benzonitrile, tertiary amines, e.g. pyridine, quinoline, picoline, lutidine and triethylamine, an example of such a PdX₂L₂ compound being dichlorobis(benzonitrile) palladium; palladium chelates such as palladium acetylacetonate; n-allyl complexes, like n-allyl palladium chloride or acetate, and bis (n-allyl) palladium; and 1,5-cyclooctadiene palladium chloride. It is noted that in case a palladium halide is used, also a catalyst activator is required, in order to dissociate the palladium halide in the solution so as to form a catalytically active species. Suitable palladium(0) complexes include a wide variety of substances containing as ligands, for example phosphines, alkenes, dienes, or nitriles. Examples of these include tetrakis(triphenylphosphine) palladium, bis(1,5-cyclooctadiene) palladium, and the like.

It is necessary to employ in step (i) of the present process, besides the Group VIII transition metal catalyst, a tertiary phosphorous ligand compound. Suitable ligand compounds comprise the substituted hydrides of phosphorous and especially those in which the hydride is fully substituted by alkyl, alkoxy, cycloalkyl, cycloalkoxy, aryl and/or aryloxy groups. Also ligand compounds in which two phosphorous atoms are attached to each other through an alkylene group can be used. Examples of the latter compounds include diphosphines.

Tertiary phosphines which may be used advantageously include phosphines containing (cyclo)alkyl and/or aryl groups. Examples of suitable trialkylphosphines are triethylphosphine, tri-n-butylphosphine, tri-isobutylphosphine, tripentylphosphine, trihexylphosphine,trioctylphosphine, trilaurylphosphine, lauryldimethylphosphine, hexyldipropylphosphine and ethylbutyloctylphosphine. Examples of suitable phosphines containing one or more aromatic groups or cycloalkyl groups are tricyclohexylphosphine, triphenylphosphine, tri(p-tolyl)phosphine, dimethylphenylphosphine, methyldiphenylphosphine, tri(m-ethylphenyl)phosphine and tri(2,4-dimethylphenyl)phosphine. A suitable diphosphine which may be used is bis(diphenylphosphine)ethane. The above-mentioned ligand compounds may in addition be substituted by halogen atoms, such as chlorine atoms, nitrile and/or nitro groups and the like. Also ligand compounds which contain in addition hydrophilic groups, such as -COOM, -SO₃M, and/or -NH₂ groups attached directly or via an alkylene group to the phosphine substituent(s), may be used. In the above-mentioned groups M represents an inorganic or organic cationic residue, such as an alkali or alkaline earth metal cation, a quarternary ammonium ion or the like.

It has been found advantageous to carry out the telomerization reaction in the presence of a basic compound (hereinafter referred to as "catalyst promoter"). Suitable catalyst promoters include alkali metal, alkaline earth metal or quaternary ammonium hydroxides, alcoholates or alkoxides, enolates, phenoxides, alkali metal borohydride, hydrazines and the like. As catalyst promoter preferably an alkali metal salt, especially the sodium or potassium salt of the active hydrogen-containing compound R-H is used. These alkali metal salts, for example, may be added as such or may be formed in situ by dissolving an alkali metal in the active hydrogen-containing compound.

The amount of palladium catalyst to be used is not critical, and any catalytically effective amount may be used. In general, amounts between 0.000001 and 0.0001 gramatom of palladium catalyst per mole of 1,3-butadiene can be used. In order to achieve high catalyst efficiencies without having to recycle the catalyst, amounts of less than 0.0001, preferably less than 0.00005, and more preferably less than 0.00002 gramatoms of palladium catalyst are used per mole of 1.3-butadiene.

The ligand compound is generally used in a relative amount of 1 to 20 moles, and preferably 2 to 15 moles of ligand compound per gramatom of the palladium catalyst. The ligand compound may be added as a separate compound to the reaction mixture or zone or to the catalyst make-up solution, or it may be incorporated in a palladium catalyst complex. When, for example, tetrakis(triphenylphosphine) palladium is used as catalyst compound, it is generally not necessary to add additional triphenylphosphine as ligand compound. The catalyst promoter is usually employed in a relative amount of 0.5 to 1,000 moles, and more preferably 0.5-150 moles of promoter per gramatom of the palladium catalyst.

The process according to the present invention should preferably be conducted in the substantial absence of oxygen, as oxygen reacts with the ligand compound and consequently may result in decreased catalyst activity.

Step (i) of the process of the present invention is conducted in the liquid phase and may be carried out in the presence or absence of a solvent which is inert under reaction conditions. When one or more of the reactants are not liquid under reaction conditions or if the reactants are poorly miscible or substantially immiscible with each other then preferably a solvent is used to secure the desired homogeneous system. Any solvent that will solubilize 1,3-butadiene, the active hydrogen-containing compound and the catalyst, ligand and optional promoter components may be used in the present process. Suitable inert solvents are (cyclo)alkanes, for example C₆₋₁₂-alkanes and cyclohexane or a mixture thereof; aromatic compounds, e.g. benzene, toluene, xylene, naphthenes; a polar solvent, such as tertiary alcohols, an amide, e.g. acetamide, dimethylacetamide or dimethylformamide; a nitrile compound, such as acetonitrile or benzonitrile; a ketone, e.g. acetone or methyl ethylketone; an ester compound, such as ethylene glycoldiacetate; an ether compound, e.g. ethylether, propylether, dioxane, tetrahydrofuran, and anisole, or a lower alkyl-disubstituted ether of mono- or diethylene glycol; and dimethylsulfoxide and sulpholane. Also water may be used as solvent or co-solvent.

The temperature at which the telomerization reaction is carried out is not critical. Normally, temperatures between ambient temperature and 150°C can be used. Preferably, the reaction temperature is 50-100°C and more preferably 70-100°C.

The pressure at which the telomerization reaction is carried out is not critical. Generally the reaction pressure lies between 1 and 40 bars, preferably between 5 and 30 bars and most preferably between 10 and 20 bars.

In general the reaction time is not critical. Reaction times of from 0.1 to 100 hours give good results, depending on reaction temperatures, reactants, catalyst system and optional solvent. Preferably reaction times lie between 0.1 and 20 hours and more preferably between 0.2 and 10 hours.

In process step (i) any type of reactor may be used. Examples of suitable reactors are a stirred tank reactor and a tubular reactor.

Step (i) of the process of the present invention generally produces a mixture of 1-substituted-2,7-octadiene, 3-substituted-1,7-octadiene and 1,3,7-octatriene, as well as a minor amount of high boiling components. Substantially no branched C₈-products are formed.

It is advantageous to recover the 1-substituted-2,7-octadiene from the reaction mixture by means of known techniques. Depending on the physical properties of reactants, catalyst components, optional solvent and the reaction products, the desired product (1-substituted-2,7-octadiene) may be isolated by distillation, extraction or other well known separation techniques, or a combination thereof. Preferably, the reaction mixture is subjected to one or more distillation steps in order to recover the 1-substituted-2,7-octadiene. The number and nature of the distillation steps will depend on the relative boiling points of the 1- and 3-substituted octadienes, the octatriene, the active hydrogen-containing compound R-H and the optional solvent. The 1,3-butadiene, and other components present in a crude C₄-hydrocarbon mixture, if used instead of pure 1,3-butadiene, have a significantly lower boiling point than the reaction products and thus can be easily separated. On the other hand, the high boiling compounds and the catalyst or catalyst decomposition products generally remain in the distillation residue.

At this point in the process the 3-substituted-1,7-octadiene and/or the 1,3,7-octatriene can advantageously also be separated from the 1-substituted-2,7-octadiene, such as for example by well known distillation techniques. It may, however, be more convenient to separate the 3-substituted-1,7-octadiene and/or 1,3,7-octatriene after they have been subjected to step (ii) of the present process, the hydrogenation step. This could be the case, for example, if the difference in the physical property relevant for the separation technique, e.g. in boiling point, between the 3-substituted octane and/or octane, which are the hydrogenation products of the 3-substituted-1,7-octadiene and 1,3,7-octatriene, and the 1-substituted octane, which is the hydrogenation product of the 1-substituted-2,7-octadiene, would be greater than the difference in the physical property relevant for the separation technique, e.g. in boiling point, between the 3-substituted-2,7-octadiene and/or 1,3,7-octatriene and the 1-substituted-1,7-octadiene.

The 1,3-butadiene, active hydrogen-containing compound R-H and optional solvent recovered can be recycled to step (i) of the process. The catalyst can be separated from the other components in the reaction mixture by means of extraction, precipitation or distillation, in which latter case it remains in the distillation residue. The catalyst in the residue or extractant may subsequently be regenerated, if desired, and recycled to step (i) or be separated from said residue or extractant, respectively, by means of precipitation, filtration, extraction or combination thereof. These catalyst recovery and recycling techniques are all well known in the art.

The 1-substituted-2,7-octadiene formed in process step (i), optionally containing minor amount of the by-products 3-substituted-1,7-octadiene and/or 1,3,7-octatriene, is subjected to hydrogenation in process step (ii) to form the 1-substituted octane of formula CH₃-CH₂-CH₂-CH₂-CH₂-CH₂-CH₂-CH₂-R, optionally together with the 3-substituted octane and/or n-octane.

In general, any conventional hydrogenation process can be used. The hydrogenation may be carried out in the liquid phase or in the vapor phase. Depending on the nature of the starting material, the reaction can be carried out at a temperature between 0 and 400°C. Preferably the temperature is in the range from ambient to 350°C. More preferably the hydrogenation is carried out at a temperature between 50 and 200°C. The pressure is not critical and depends on whether the hydrogenation is carried out in the liquid or vapor phase. In general the pressure can vary between 0.1 and 100 bar.

In process step (ii) any of the conventional homogeneous and heterogeneous hydrogenation catalysts can be used. Examples of such catalysts include the Group VIII of the periodic system transition metal catalysts. Preferred catalysts are the noble metal catalysts of this group VIII, either in elemental form or in the form of a compound which is reducible by hydrogen under hydrogenation conditions to form finely divided elemental metal, or mixtures thereof. Examples of such compounds are the oxides, halides, nitrates, oxalates, acetates, carbamates, propionates, tartrates, hydroxides and the like or mixtures thereof. Preferred catalysts are palladium and platinum, whereas also Raney-nickel may advantageously be used. These catalysts may be modified with other metals. The catalysts may preferably be supported on a carrier material, such as for example active carbon, alumina, silica, silica-alumina, calcium carbonate, barium carbonate, barium sulfate, clays, Kieselguhr and the like. Particularly preferred catalysts are alumina supported platinum or palladium catalysts, the metal preferably being dispersed on the support in an amount of 0.1 to 1.0 wt%.

The hydrogenation catalysts are used in the amounts conventional for these type of reactions and catalysts.

Generally, the relative amounts of hydrogen, in the form of pure hydrogen or as a hydrogen containing gas, and the 1-substituted-2,7-octadiene are not critical, but it is preferred to use a molar ratio between hydrogen and the 1-substituted-2,7-octadiene in the range from 2:1 to 50:1, more preferably from 2:1 to 10:1.

The hydrogenation can advantageously be carried out in a diluent or solvent for dilution of the material to be hydrogenated. The usual diluents may be used for this, such as saturated hydrocarbons having 4 to 12 carbon atoms, alkanols containing from 1 to 12 carbon atoms, acyclic and cyclic ethers having 4 to 12 carbon atoms and mixtures thereof. Advantageously, part of the reaction product of the hydrogenation reaction is recycled to step (ii) and used as diluent in the hydrogenation reaction.

The hydrogenation in step (ii) can be carried out according to batch, semi-continuous or continuous procedures. It may also be performed in more than one step, such as for example in a preliminary hydrogenation step and a finishing hydrogenation step. Typical weight hourly space velocities (based on reactant) are in the range of 0.1-50, preferably in the range of 0.2-10 and more preferably in the range of 0.5-5.

The 1-substituted-2,7-octadiene formed in process step (i), optionally containing 3-substituted-1,7-octadiene and/or 1,3,7-octatriene is hydrogenated in step (ii) of the present process at very high conversion and selectivity. Apart from the reaction product 1-substituted octane and, depending on the feed composition, 3-substituted octane and n-octane, minor amounts of 1-octene and its internal linear isomers may be formed due to decomposition of the 1- or 3-substituted octanes under hydrogenation conditions. Accordingly, both the telomerization and hydrogenation products obtained by the process of the present invention contain substantially no branched C₈-isomers.

Depending on the physical properties of the starting compound(s), catalyst, optional diluent and the reaction product(s), the desired product (1-substituted octane) may be isolated by distillation, extraction or other well known separation techniques, or a combination thereof. The reaction mixture may advantageously be subjected to one or more distillation steps in order to recover the 1-substituted octane. The number and nature of the distillation steps will depend on the relative boiling points of the 1- and 3-substituted octanes, the n-octane and the optional solvent.

It is a matter of convenience and it will depend on the physical properties of the components present in the hydrogenation reaction mixture, whether the desired reaction product (1-substituted octane) is recovered in a substantially pure form (>99%) and as such used in step (iii) of the present process, or alternatively, a mixture of reaction products is used in the last process step. The latter would be preferred, for example, if the difference in the physical property relevant for the separation technique, e.g. in boiling point, between the 3-substituted octane on the one hand, and the 1-substituted octane, on the other hand, would be smaller than the difference in the relevant physical property, e.g. in boiling point, between the decomposition (in step (iii)) products of 3-substituted octane, on the one hand, and 1-octene, on the other hand. At the high conversions and selectivities, apart from the removal of unconsumed hydrogen, and optional solvent, no further purification of the 1-substituted octane is necessary if the 1-substituted-2,7-octadiene is subjected to the hydrogenation step in a pure form (≧99%)

Generally, it is preferred to purify the 1-substituted octane as much as possible, so that the amount of 3-octene, which is one of the decomposition products of the 3-substituted octane, is minimal, as 3-octene will be relatively difficult to separate from 1-octene.

The catalyst can be separated (if not present in a fixed bed) from the reaction mixture by extraction, distillation or conventional filtration means. The reactants and/or diluent can be recycled to the hydrogenation reaction, if desired. As mentioned before, it is advantageous to recycle the reaction product in order to serve as diluent.

It is noted that the group R in the 1-substituted-2,7-octadiene compound to be hydrogenated can also be a group which can be hydrogenated under the prevailing hydrogenation conditions, provided the thus hydrogenated group R is readily split off in the last step of the process to yield 1-octene.

According to process step (iii) of the present invention, the 1-substituted octane of formula CH₃-CH₂-CH₂-CH₂-CH₂-CH₂-CH₂-CH₂-R formed in step (ii) is subsequently decomposed in the presence of a suitable catalyst to form 1-octene.

The type of decomposition reaction which is the subject of process step (iii) is in itself well known in the art. In process step (iii) of the present invention any suitable conventional catalyst can be used which is capable of decomposing the 1-substituted octane to give 1-octene. Generally, a solid acidic catalyst is used for this purpose. Preferably an alumina catalyst is used. Examples of such catalysts are alpha, delta, gamma, etha and theta-aluminas, which may be modified with metal-containing weakly acidic components, organic carboxylic acids, or other treating agents.

The temperatures at which the decomposition process is carried out depend on the decomposition temperature of the respective compound and on the catalyst activity. Generally the temperatures are up to 500°C, preferably in the range of 200 to 400°C, whereas temperatures in the range of 250 to 350°C are more preferred.

The pressure under which the decomposition reaction may be carried out is not critical and can vary widely, for example from 0.1 to about 50 bar. Preferably the reaction is carried out at a pressure between 0.2 and 10 bar.

The reaction can be carried out in the vapor or in the liquid phase, the vapor phase being preferred. An inert carrier gas or an inert diluent may be used to dilute the reactant. Examples of such inert gases are nitrogen, helium, argon and the like or mixtures thereof.

The reaction may be carried out continuously, semi-continuously or batchwise. In the continuous mode the reactant(s) and possibly the diluent are continuously passed over a bed of the catalyst at the desired reaction conditions. The reactant is generally added to the reactor containing the catalyst under the desired reaction conditions at a weight hourly space velocity of about 0.01 to about 50, and preferably about 0.1 to about 10.

The desired product 1-octene may be recovered from the reaction mixture using conventional methods such as solvent extraction, (fractional) distillation, (fractional) condensation etc. The unconverted reactant(s) can be recycled to this reaction step (iii). The other product of the decomposition reaction, the compound R-H can be recovered and recycled to process step (i).

Apart from 1-octene, the 1-substituted octane may also yield some 2-octene caused by isomerization. The 3-substituted octane will be decomposed to 2-octene and 3-octene, whereas any n-octane present in the feed will be inert. Minor amounts of 4-octenes may be formed due to isomerization of 3-octenes. Accordingly, the decomposition product mixture contains 1-octene and minor quantities of 2-, 3- and 4-octenes and n-octane; no branched C₈-olefin isomers will be formed.

By means of well known distillation techniques the C₈-olefinic fraction, usually containing some n-octane, unconverted 1-substituted octane and optionally unconverted 3-substituted octane can be recovered. This C₈-olefinic fraction may be subjected to a further distillation step to remove unconverted 1- and 3-substituted octanes. This already can yield a 1-octene product having a purity of about 90-97%. Finally, in a further fractional distillation step the linear internal octene isomers and n-octane can be removed so as to give 1-octene in a highly pure form (>97%). The 1-octene thus obtained contains no branched C₈-olefin isomers. The typical conditions for the separation steps, and in particular the distillation steps are largely determined by the physical properties, such as in particular the boiling points of the compounds to be separated.

The invention will be illustrated by means of the following example which by no means should be construed to limit the invention.

### Example:

A. To a tubular reactor provided with a jacket for temperature control and maintained at a pressure of 14 bar and at a temperature of 80°C, 1.3 kg/h of deoxygenated methanol, 0.8 kg/h of 1,3-butadiene, 45 g/h of a catalyst solution prepared by dissolving 18 g of palladium acetylacetonate (59 mmole) and 31 g of triphenylphosphine (118 mmole) in 16 l of deoxygenated methanol, and 30 g/h of a promoter solution containing 1.5 wt% of sodium methoxide in deoxygenated methanol were fed. In the feed stream to the reactor the methanol/1,3-butadiene ratio was 3 mole/mole, the palladium concentration was 0.000015 gram atoms per mole of 1,3-butadiene, the triphenylphosphine/palladium ratio was 2 mole/gram atom, and the sodium methoxide/palladium ratio was 100 mole/gram atom. The average residence time in the tubular reactor was 3.5 h. Gas chromatographic analysis of the reaction mixture leaving the reactor showed that 95.7% of the 1,3-butadiene fed was converted, whereas the selectivity of the converted 1,3-butadiene to (substituted) C₈ products was: 89.4% 1-methoxy-2,7-octadiene, 5.6% 3-methoxy-1,7-octadiene, and 5.0% 1,3,7-octatriene.
   The reaction mixture thus obtained was subjected to a first distillation at 1.2 bar to remove the lower boiling components 3-methoxy-1,7-octadiene, 1,3,7-octatriene, unreacted methanol and 1,3-butadiene. The bottom stream of the first distillation was subjected to a further distillation at 0.5 bar to remove 1-methoxy-2,7-octadiene from the heavier boiling components to obtain 1-methoxy-2,7-octadiene in a purity of 99.3%.
B. The 1-methoxy-2,7-octadiene having a purity of 99.3% obtained in process step (i) was fed to a tubular reactor containing 67 g of catalyst in a fixed bed at a rate of 60 g (0.43 mole) per hour (weight hourly space velocity = 0.9). The catalyst used was a palladium on alumina catalyst, containing 0.3 wt% palladium supported on γ-alumina. Hydrogen was fed to the reactor at such a rate as to maintain a molar ratio of hydrogen to 1-methoxy-2,7-octadiene of about 7:1. The temperature of the reactor was maintained at 80°C and the pressure at 15 bar to maintain a liquid phase. At the end of the reactor analysis of the reaction mixture by gas chromatography showed that the purity of the product 1-methoxyoctane in the liquid phase was 99.2% as determined by gas chromatography.
C. The 1-methoxyoctane having a purity of 99.2% obtained in process step (ii) was subjected to a decomposition reaction. The 1-methoxyoctane was fed to a preheater at a rate of 80 g (0.55 mole) per hour in order to vaporize the 1-methoxyoctane. Subsequently, the vaporized 1-methoxyoctane was fed to a tubular reactor containing 13 g of a γ-alumina catalyst in a fixed bed (weight hourly space velocity = 6.2). The temperature of the reactor was maintained at 330°C and the pressure at 1 bar absolute. At the end of the reactor, analysis of the product by gas chromatography showed that the conversion of 1-methoxyoctane was 80% and the selectivity to octenes was 66% and the selectivity to 1-octanol and dioctylether was 34%. Based on octenes present in the reaction mixture, there was 95.7% of 1-octene. The reaction mixture was subjected to an atmospheric distillation to remove methanol and other components of boiling points lower than the octenes. In a second distillation (at 0.5 bar pressure) the unconverted 1-methoxyoctane, 1-octanol and dioctylether were removed to yield an octene fraction consisting of 95.7 wt% 1-octene, 3.8 wt% 2-octenes and 0.5 wt% 3- and 4-octenes. This octene fraction was subjected to a final distillation step at a pressure of 0.5 bar to give an octene fraction of 99.0 wt% 1-octene, 0.55 wt% 2-octenes and 0.45 wt% 3- and 4-octenes.

## Claims

1. Process for producing 1-octene which comprises the steps of:
(i) reacting 1,3-butadiene with a primary aliphatic alcohol or aromatic hydroxy compound having formula R-H, in the presence of a telomerization catalyst comprising palladium and a tertiary phosphorous ligand compound, in an amount of less than 0.0001 gramatoms palladium catalyst per mole of 1,3-butadiene, to form a 1-substituted-2,7-octadiene of formula CH₂=CH-CH₂-CH₂-CH₂-CH=CH-CH₂-R, in which R represents the residue of the primary aliphatic alcohol or aromatic hydroxy compound;
(ii) subjecting the 1-substituted-2,7-octadiene formed in step (i) to hydrogenation in the presence of a hydrogenation catalyst to form a 1-substituted octane of formula CH₃-CH₂-CH₂-CH₂-CH₂-CH₂-CH₂-CH₂-R; and
(iii) decomposing the 1-substituted octane formed in step (ii) in the presence of a suitable catalyst to form 1-octene.

2. Process according to Claim 1, wherein in step (i) as primary aliphatic alcohol methanol or ethanol is used.

3. Process according to any one of the Claims 1-2, wherein in step (i) the reaction is carried out in the presence of a catalyst promotor.

4. Process according to Claim 3, wherein in step (i) as catalyst promotor an alkali metal salt of the active hydrogen-containing compound R-H is used.

5. Process according to any one of the Claims 1-4, wherein in step (ii) the hydrogenation is carried out in a diluent at a temperature between 0 and 400°C in the presence of a hydrogenation catalyst, and the molar ratio of hydrogen to 1-substituted-2,7-octadiene is in the range from 2:1 to 50:1.

6. Process according to Claim 5, wherein in step (ii) the hydrogenation catalyst is a supported platinum or palladium catalyst.

7. Process according to Claims 5 or 6, wherein part of the reaction product of the hydrogenation reaction is recycled to step (ii) and used as diluent in the hydrogenation reaction.

8. Process according to any one of the Claims 1-7, wherein in step (iii) the decomposition reaction is carried out in the vapor phase at a temperature up to 500°C over a fixed bed of an alumina catalyst.

## Patentansprüche

1. Verfahren zum Herstellen von 1-Octen, umfassend die Schritte:
(i) Umsetzen von 1,3-Butadien mit einem primären aliphatischen Alkohol oder einer aromatischen Hydroxy-Verbindung der Formel R-H in der Gegenwart eines Palladium und eine Verbindung mit einem tertiären Phosphorliganden enthaltenden Telomerisierungskatalysators, in einer Menge von wenigor als 0,0001 Grammatom Palladium-Katalysator pro Mol 1,3-Butadien, um ein 1-substituiertes 2,7-Octadien der Formel CH₂=CH-CH₂-CH₂-CH₂-CH=CH-CH₂-R zu bilden, worin R den Rest des primären aliphatischen Alkohols oder der aromatischen Hydroxyverbindung bedeutet,
(ii) Unterwerfen des in Schritt (i) gebildeten 1-substituierten 2,7-Octadiens einer Hydrierung in der Gegenwart eines Hydrierungskatalysators, um ein 1-substituiertes Octan der Formel CH₃-CH₂-CH₂-CH₂-CH₂-CH₂-CH₂-CH₂-R zu bilden, und
(iii) Zersetzen des in Schritt (ii) gebildeten 1-substituierten Octans in der Gegenwart eines geeigneten Katalysators, um 1-Octen zu bilden.

2. Verfahren nach Anspruch 1, worin in Schritt (i) als primärer aliphatischer Alkohol Methanol oder Ethanol verwendet wird.

3. Verfahren nach einem der Ansprüche 1-2, worin in Schritt (i) die Reaktion in Gegenwart eines Katalysator-Promotors durchgeführt wird.

4. Verfahren nach Anspruch 3, worin in Schritt (i) als Katalysator-Promotor ein Alkalimetallsalz der aktiven Wasserstoff enthaltenden Verbindung R-H verwendet wird.

5. Verfahren nach einem der Ansprüche 1-4, worin in Schritt (ii) die Hydrierung in einem Verdünnungsmittel bei einer Temperatur zwischen 0 und 400 °C in der Gegenwart eines Hydrierungskatalysators durchgeführt wird und worin das molare Verhältnis von Wasserstoff zu 1-substituiertem 2,7-Octadien im Bereich von 2 : 1 bis 50 : 1 ist.

6. Verfahren nach Anspruch 5, worin in Schritt (ii) der Hydrierungskatalysator ein Platin- oder Palladium-Trägerkatalysator ist.

7. Verfahren nach Anspruch 5 oder 6, worin ein Teil des Reaktionsprodukts der Hydrierungsreaktion in Schritt (ii) zurückgeführt und als Verdünnungsmittel in der Hydrierungsreaktion verwendet wird.

8. Verfahren nach einem der Ansprüche 1-7, worin in Schritt (iii) die Zersetzungsreaktion in der Dampfphase bei einer Temperatur von bis zu 500 °C über einem Festbett von einem Aluminiumoxidkatalysator durchgeführt wird.

## Revendications

1. Procédé pour produire du 1-octène qui comprend les étapes consistant:
(i) à faire réagir du 1,3-butadiène avec un alcool aliphatique primaire ou un composé hydroxyaromatique ayant la formule R-H, en présence d'un catalyseur de télomérisation, comprenant du palladium et un composé ligand de phosphore tertiaire, en une quantité inférieure à 0,0001 atome-gramme de catalyseur à base de palladium par mole de 1,3-butadiène, pour former un 2,7-octadiène substitué en 1 de formule CH₂=CH-CH₂-CH₂-CH₂-CH=CH-CH₂-R, dans lequel R représente le résidu de l'alcool aliphatique primaire ou du composé hydroxyaromatique;
(ii) à soumettre le 2,7-octadiène substitué en 1 formé dans l'étape (i) à une hydrogénation en présence d'un catalyseur d'hydrogénation pour former un octane substitué en 1 de formule CH₃-CH₂-CH₂-CH₂-CH₂-CH₂-CH₂-CH₂-R;
(iii) à décomposer l'octane substitué en 1 formé dans l'étape (ii) en présence d'un catalyseur approprié pour former le 1-octène.

2. Procédé selon la revendication 1, dans lequel dans l'étape (i), en tant qu'alcool aliphatique primaire, on utilise le méthanol ou l'éthanol.

3. Procédé selon l'une quelconque des revendications 1 et 2, dans lequel dans l'étape (i) la réaction est effectuée en présence d'un activateur catalytique.

4. Procédé selon la revendication 3, dans lequel dans l'étape (i), en tant qu'activateur catalytique, on utilise un sel de métal alcalin du composé R-H contenant de l'hydrogène actif.

5. Procédé selon l'une quelconque des revendications 1 à 4, dans lequel dans l'étape (ii), l'hydrogénation est effectuée dans un diluant à une température comprise entre 0 et 400°C en présence d'un catalyseur d'hydrogénation, et le rapport molaire de l'hydrogène au 2,7-octadiène substitué en 1 est compris entre 2:1 et 50:1.

6. Procédé selon la revendication 5, dans lequel dans l'étape (ii), le catalyseur d'hydrogénation est un catalyseur à base de palladium ou de platine sur support.

7. Procédé selon la revendication 5 ou 6, dans lequel une partie du produit réactionnel de la réaction d'hydrogénation est recyclée vers l'étape (ii) et est utilisée en tant que diluant dans la réaction d'hydrogénation.

8. Procédé selon l'une quelconque des revendications 1 à 7, dans lequel dans l'étape (iii), la réaction de décomposition est effectuée en phase vapeur à une température s'élevant jusqu'à 500°C sur un lit fixe d'un catalyseur à base d'alumine.
